# EUROPÄISCHE PATENTSCHRIFT

(11) **EP 2 705 063 B1**
(45) Veröffentlichungstag und Bekanntmachung des Hinweises auf die Patenterteilung: **12.07.2017**
(21) Anmeldenummer: 12717296.3
(22) Anmeldetag: 27.04.2012
(51) Int. Cl.: C08G 18/02, C07C 267/00, C08K 5/29

(54) **CARBODIIMIDE AUS TRISUBSTITUIERTEN AROMATISCHEN ISOCYANATEN, EIN VERFAHREN ZU DEREN HERSTELLUNG UND DEREN VERWENDUNG**
CARBODIIMIDES FROM TRISUBSTITUTED AROMATIC ISOCYANATES, A METHOD FOR PRODUCING SAME, AND THE USE OF SAME
CARBODIIMIDES OBTENUS À PARTIR D'ISOCYANATES AROMATIQUES TRISUBSTITUÉS, LEUR PROCÉDÉ DE PRODUCTION ET LEUR UTILISATION

(30) Priorität: 05.05.2011 EP 11164899; 09.11.2011 EP 11188364
(43) Veröffentlichungstag der Anmeldung: 12.03.2014
(73) Patentinhaber: LANXESS Deutschland GmbH, 50569 Köln (DE)
(72) Erfinder: LAUFER, Wilhelm, 67158 Ellerstadt (DE); BECHEM, Benjamin, 68157 Mannheim (DE); ECKERT, Armin, 68794 Oberhausen (DE)
(86) Internationale Anmeldenummer: PCT/EP2012/057708
(87) Internationale Veröffentlichungsnummer: WO 2012/150181

(56) Entgegenhaltungen:
- EP-A2- 0 450 439
- DE-A1- 2 020 330
- US-A- 3 193 522
- US-A- 3 711 439

## Beschreibung

Die Erfindung betrifft neuartige Carbodiimide, ein Verfahren zu deren Herstellung und deren Verwendung als Stabilisator, Vernetzer und/oder Kompatibilisator in Thermoplasten, esterbasierten Polyolen für Polyurethan-Anwendungen, in Hartschaum, in Weichschaum oder z.B. in CASE-Anwendungen (Coatings Adhesives Sealants Elastomers).

Carbodiimide haben sich in vielen Anwendungen bewährt, z.B. als Hydrolyseschutzmittel für Thermoplasten, Polyolen, Polyurethane etc.

Bevorzugt werden hierfür sterisch gehinderte Carbodiimide eingesetzt. Bekannt sind in diesem Zusammenhang vor allem das Bis-2,6-Diisopropylphenyl-Carbodiimid und/oder Carbodiimide auf Basis 2,4,6-Triisopropylphenyl-1,3-diisocyanat oder auf Basis Tetramethylxylylendiisocyanat, erhältlich bei der Rhein Chemie Rheinau GmbH. Polycarbodiimide aus 1,3,5-Triisopropyl-2,4-disocyanatobenzol und 2,6-Diisopropylphenylisocyanat mit zweifach substituierten endständigen Isocyanatresten sind aus der EP 0 450 439 A bekannt. Die im Stand der Technik bekannten Carbodiimide haben jedoch die Nachteile in bestimmten Anwendungen, z. B. bei der Folienherstellung bei höheren Temperaturen, flüchtig oder thermisch nicht stabil zu sein und können flüchtige giftige Verbindungen abspalten und/oder müssen in der Regel aufgrund der niedrigeren Funktionalität unwirtschaftlich in hohen Mengen dosiert werden.

Es bestand daher ein Bedarf an Carbodiimiden, welche die vorgenannten Nachteile nicht aufweisen.

Aufgabe der vorliegenden Erfindung war daher die Bereitstellung von Carbodiimiden, welche temperaturstabil und wirtschaftlich herstellbar sind.

Überraschenderweise konnte diese Aufgabe durch bestimmte Carbodiimide gelöst werden.

Gegenstand der vorliegenden Erfindung sind daher Carbodiimide der Formel (I) 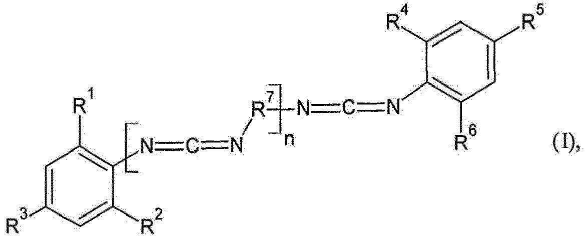 in der R¹, R², R³, R⁴, R⁵ und R⁶ unabhängig voneinander C₁-C₂₀-Alkyl, C₃-C₂₀-Cycloalkyl, C₆-C₁₅-Aryl und/oder C₇-C₁₅-Aralkyl ist,
R⁷ = C₁ - C₁₈-Alkylen, C₅-C₁₈-Cycloalkylen-, alkyl-substituiertes Arylen und/oder C₇-C₁₈-Aralkylen , bevorzugt Alkyl-substituiertes Arylen und/oder C₇-C₁₈- Aralkylen und n eine ganze Zahl von 1 bis 500 ist. Vorzugsweise liegt n zwischen 1 und 50, ganz besonders bevorzugt von 4 - 50.

In einer Ausführungsform der Erfindung können auch Mischungen der Verbindungen der Formel (I) auftreten. Im Fall der Mischung können sich bei der Ermittlung des Mittelwertes für n auch gebrochenen Zahlen ergeben.Bei dem Alkyl-substituiertes Arylen handelt es sich vorzugsweise um C₁-C₄-alkyl-substituiertes Arylen, besonders bevorzugt um ein- bis dreifach substituiertes C₁-C₄-alkyl-substituiertes Arylen, ganz besonders bevorzugt Triisopropylphenylen.

Bei dem C₇-C₁₈- Aralkylen handelt es sich vorzugsweise um Tetramethylxylylen.Die Alkylreste sind vorzugsweise verzweigt. In einer Ausführungsform der Erfindung sind die C₃-C₂₀-Alkylreste dabei linear und/oder verzweigt.Bei den erfindungsgemäßen Carbodiimiden der Formel (I) sind die Reste R¹ bis R⁶ vorzugsweise gleich.In einer weiteren bevorzugten Ausführungsform der Erfindung entsprechen die Reste R¹ bis R⁶ Isopropyl. Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die Verbindungen der Formel (I) sind thermisch stabil und zeichnen sich durch eine hervorragende Wirksamkeit als Hydrolyseschutzmittel/Säurefänger in estergruppen-haltigen Polymeren aus.

Gegenstand der vorliegenden Erfindung ist zudem ein Verfahren zur Herstellung der erfindungsgemäßen Carbodiimide, wonach trisubstituierte Phenylisocyanate der Formel (II) 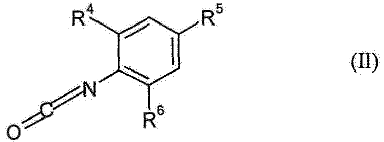 und der Formel (III)
R⁷ = C₁ - C₁₈-Alkylen, C₅-C₁₈-Cycloalkylen-, alkyl-substituiertes Arylen und/oder C₇-C₁₈-Aralkylen , bevorzugt Alkyl-substituiertes Arylen und/oder C₇-C₁₈- Aralkylen und n eine ganze Zahl von 4 bis 500 ist. Vorzugsweise liegt n zwischen 4 - 50.

In einer Ausführungsform der Erfindung können auch Mischungen der Verbindungen der Formel (I) auftreten. Im Fall der Mischung können sich bei der Ermittlung des Mittelwertes für n auch gebrochenen Zahlen ergeben.

Bei dem Alkyl-substituiertes Arylen handelt es sich vorzugsweise um C₁-C₄-alkyl-substituiertes Arylen, besonders bevorzugt um ein- bis dreifach substituiertes C₁-C₄-alkyl-substituiertes Arylen, ganz besonders bevorzugt Triisopropylphenylen.

Bei dem C₇-C₁₈- Aralkylen handelt es sich vorzugsweise um Tetramethylxylylen.

Die Alkylreste sind vorzugsweise verzweigt. In einer Ausführungsform der Erfindung sind die C₃-C₂₀-Alkylreste dabei linear und/oder verzweigt.

Bei den erfindungsgemäßen Carbodiimiden der Formel (I) sind die Reste R¹ bis R⁶ vorzugsweise gleich.

In einer weiteren bevorzugten Ausführungsform der Erfindung entsprechen die Reste R¹ bis R⁶ Isopropyl.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die Verbindungen der Formel (I) sind thermisch stabil und zeichnen sich durch eine hervorragende Wirksamkeit als Hydrolyseschutzmittel/Säurefänger in estergruppen-haltigen Polymeren aus.

Gegenstand der vorliegenden Erfindung ist zudem ein Verfahren zur Herstellung der erfindungsgemäßen Carbodiimide, wonach trisubstituierte Phenylisocyanate der Formel (II) 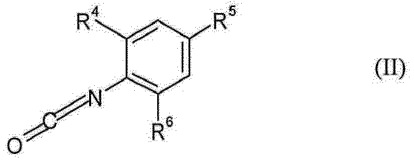 und der Formel (III) 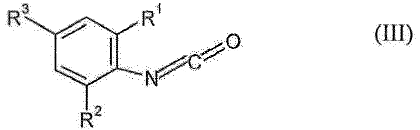 in der R¹, R², R³, R⁴, R⁵ und R⁶ unabhängig voneinander C₁-C₂₀-Alkyl, C₃-C₂₀-Cycloalkyl, C₆-C₁₅-Aryl und/oder C₇-C₁₅-Aralkyl ist
und Verbindungen der Formel (IV) III) 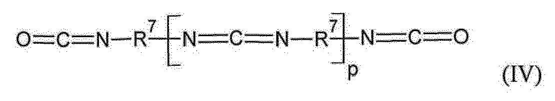 in der R⁷ = C₁-C₁₈-Alkylen, C₅-C₁₈-Cycloalkylen-, alkyl-substituiertes Arylen, C₇-C₁₈- Aralkylen, bevorzugt Alkyl-substituierten Arylen und/oder C₇-C₁₈- Aralkylen ist, und p ≥ 2 ist,
unter Abspaltung von Kohlendioxid bei Temperaturen von 40 °C bis 200 °C in Gegenwart von Katalysatoren und gegebenenfalls Lösungsmittel carbodiimidisiert werden.

Bei dem Alkyl-substituierten Arylen handelt es sich vorzugsweise um C₁-C₄-alkyl-substituiertes Arylen, besonders bevorzugt um ein- bis dreifach substituiertes C₁-C₄-alkyl-substituiertes Arylen, ganz besonders bevorzugt Triisopropylphenylen und/oder Tetramethylxylyten.

Bei den trisubstituierten Phenylisocyanaten handelt es sich vorzugsweise um Isocyanate wie 2,4,6-Triisopropylphenylisocyanat, 2,6-Diisopropyl-4-*tert*-butylphenylisocyanat, 2,6-Diisopropyl-4-stearylphenylisocyanat, 2,6-Diisopropyl-4-dodecylphenylisocyanat, 2,4,6-Tri-*tert*-butyl-phenylisocyanat, 2,4,6-Tri-2-ethylhexylphenylisocyanat, 2,6-Diisopropyl-4-*n*-butylphenylisocyanat, 2,4,6-Tri-*n*-butylphenylisocyanat, 2,4,6-Tri-hexadecanylphenylisocyanat und/oder 2,4,6-Trioctadecanylphenylisocyanat.

Die trisubstituierten Phenylisocyanate können ausgehend von trisubstituierten Anilinen hergestellt werden.

Diese trisubstituierten Aniline können - wie dem Fachmann bekannt- über eine Friedel Crafts Alkylierung von Anilin mit dem entsprechenden Alken, Halogenalkan, Halogenalkenbenzol und/oder Halogencycloalkan hergestellt werden. Die Diarylanilin-Derivate können alternativ auch z.B. ausgehend von dem 2,6-Dibromanilin mittels Suzuki-Kupplung synthetisiert werden.

Anschließend werden diese trisubstituierten Aniline mit Phosgen zum entsprechenden trisubstituierten Phenylisocyanat umgesetzt. Die eingesetzten trisubstituierten Aniline sind auch kommerziell erhältlich, z.B. bei der Firma Lonza Group Ltd.

Bei den Verbindungen der Formel (IV) handelt es sich um handelsübliche Stoffe, die z.B. bei der Firma Rhein Chemie Rheinau GmbH z.B. unter dem Handelsnamen Stabaxol® P 220, Stabaxol® P 100 oder z.B. bei der Firma Bayer MaterialScience AG z.B. unter dem Handelsnamen Desmodur® W, Desmodur® I, Desmodur® 44 M und Desmodur® T erhältlich sind.

Die Carbodiimidisierung erfolgt dabei vorzugsweise gemäß der in Angew. Chem. 93, S. 855 - 866 (1981) oder DE-A-11 30 594 oder Tetrahedron Letters 48 (2007), S. 6002 - 6004 beschriebenen Verfahren.

Als Katalysatoren sind in einer Ausführungsform der Erfindung starke Basen oder Phosphorverbindungen bevorzugt. Vorzugsweise werden Phospholenoxide, Phospholidine oder Phospholinoxide sowie die entsprechenden Sulfide verwendet. Ferner können als Katalysatoren tertiäre Amine, basisch reagierende Metallverbindungen, Alkali-, Erdalkalioxide oder -Hydroxide, - Alkoholate oder -Phenolate, Carbonsäuremetallsalze und nicht basische Organometallverbindungen verwendet werden.

Die Carbodiimidisierung kann sowohl in Substanz als auch in einem Lösemittel durchgeführt werden. In einer weiteren Ausführungsform der Erfindung wird die Carbodiimidisierung zuerst in Substanz und anschließend im Lösemittel durchgeführt. Als Lösemittel können z. B. Benzine, Benzol und/oder Alkylbenzole eingesetzt werden.

Die erhaltenen erfindungsgemäßen Carbodiimide können gegebenenfalls nach der Reaktion gereinigt werden. Die Reinigung der Rohprodukte kann mittels Umkristallisation erfolgen. Als geeignete Lösemittel für das Umkristallisieren können z. B. Alkylbenzole, Alkohole, Ketone, Ether oder Ester eingesetzt werden.

Gegenstand der vorliegenden Erfindung ist zudem die Verwendung der erfindungsgemäßen Carbodiimide als Stabilisator, Vernetzer und/oder Kompatibilisator in Thermoplasten, wie z.B. Polyethylenterephthalat (PET), Polybutylenterephthalat (PBT), Polytrimethylenterephthalat (PTT), in Thermoplastischen Polyurethanen (TPU), Copolyestern, wie das modifizierte Polyester aus Cyclohexandiol und Terephthalsäure (PCTA), in thermoplastischen Polyester Elastomeren (TPE E), Polymilchsäure (PLA) und/oder PLA-Derivaten, Polyhydroxyalkanoaten (PHA), stärkebasierten Kunststoffen, Polyamiden (PA), wie z.B. Polyamid 6, 6.6, 6.10, 612. 10, 11, 12, oder in Blends, wie z.B. PA/PET- oder PHA/PLA-Blends.

Gegenstand der Erfindung ist zudem die Verwendung der erfindungsgemäßen Carbodiimide als Hydrolyseschutzmittel in esterbasierten Polyolen, wie z.B. petrochemisch oder biobasierten Polyolen, für Polyurethan-Hart- und Weichschäume, estergruppen-enthaltende Polyurethanklebstoffe, Schmierstoffe und/oder Öle, wie z.B. in Transformatorenölen, und/oder für Polyurethan-basierte CASE (Coatings Adhesives Sealants Elastomers)- Anwendungen.

Gegenstand der vorliegenden Erfindung ist zudem die Verwendung der erfindungsgemäßen Carbodiimide in fester Form vorzugsweise zur Feststoffdosierung auf kontinuierlich arbeitenden Verarbeitungsmaschinen, wie z.B. Ein-, Zwei- und Mehrwellenextrudern, kontinuierlich arbeitenden Co-Knetern (Typ Buss) und diskontinuierlich arbeitenden Knetern, z.B Typ Banbury und anderen in der Polymerindustrie üblichen Aggregaten.

Gegenstand der vorliegenden Erfindung ist zudem die Verwendung der erfindungsgemäßen Carbodiimide bei der Herstellung von Kunststofffolien, insbesondere PET-Folien, TPU-Folien und PLA-Folien.

Die nachfolgenden Beispiele dienen der Erläuterung der Erfindung, ohne dabei limitierend zu wirken.

### Ausführungsbeispiele

Es wurde ein Carbodiimid auf Basis von Tetramethylxylylendiisocyanat, umgesetzt mit Polyethylenglykolmonomethylether, erhältlich unter der Bezeichnung Stabaxol ® P 200 sowie ein Bis-2,6-Diisopropylphenyl-Carbodiimid (Stabaxol^{®} I) der Firma Rhein Chemie Rheinau GmbH und ein heteropolymeres Carbodiimid auf Basis Triisopropylphenyldiisocyanat und 2,6-Diisopropylphenylisocyanat (CDI 0) im Vergleich zum erfindungsgemäßen Carbodiimid I auf Basis Tetramethylxylylendiisocyanat, umgesetzt mit Triisopropylphenylisocyanat sowie ein Carbodiimid II auf Basis von Triisopropylphenyldiisocyanat und Triisopropylphenylisocyanat getestet.

Die vorgenannten Carbodiimide wurden bezüglich der Hydrolyseschutz/Säurezahlabbau-Wirkung in esterbasierten Polymeren geprüft.

### Herstellung der erfindungsgemäßen Carbodiimide I und II

In einem ausgeheizten und mit Stickstoff befüllten 500 ml Planschliffkolben wurden unter Stickstoffstrom 400 g Carbodiimid auf Basis Tetramethylxylylendiisocyanat (für Carbodiimid I) bzw. auf Basis Triisopropylphenyldiisocyanat (für Carbodiimid II) und 210 g 2,4,6-Triisopropylphenylisocyanat vorgelegt und auf 140 °C erwärmt. Nach Zugabe von 400 mg 1-Methyl-phospholenoxid wurde das Reaktionsgemisch innerhalb von 5 Stunden auf 180 °C erhitzt. Im Anschluss wurde bei 180 °C solange umgesetzt bis ein NCO-Gehalt von < 1 % erreicht worden ist.

### Anwendungstests

### Thermische Stabilität

Zur Ermittlung der thermischen Stabilität wurden thermogravimetrische Analysen mit einer TGA-Messeinrichtung der Firma Mettler Toledo (TGA/SDTA851) durchgeführt. Dafür wurden je 10 - 15 mg Probe unter Stickstoff in einer Temperaturrampe von 30 bis 600 °C und bei einer Heizrate von 10 °C / min analysiert. Bewertet wurde die Temperatur in °C beim Erreichen des Gewichtverlustes von 10 %.

### Hydrolysestabilität von Polyethylenterephthalat

Zur Bestimmung der Hydrolysestabilität wurden PET-Prüfkörper mit 1,5 Gew. % Carbodiimid nach der Hydrolysealterung bei 120 °C im Sattdampf auf Zugfestigkeit untersucht. Bewertet wurde die Dauer in Tage bis die Zugfestigkeit den Wert 0 erreicht hat. Als weiterer Vergleich wurden auch das PET getestet, das nicht mit Carbodiimid stabilisiert wurde.

Die Ergebnisse sind in Tabelle 1 dargestellt:

**Tabelle 1:**

| Carbodiimid | TGA von Carbodiimid [°C] | Hydrolysestabilität von PET [Tage] |
|---|---|---|
| Kein Carbodiimid (V) | - | 2-3 |
| Stabaxol^{®} I(V) | 240 | 7 |
| Stabaxol^{®} P 200 (V) | 300 | 4-5 |
| CDI 0 (V) | 320 | 6-7 |
| CDI I (V) | 300 | 7 |
| CDI II (erf) | 360 | 6-7 |

| | | |
|---|---|---|
| V = Vergleichsbeispiel, erf = erfindungsgemäß | | |

### Testergebnisse

Die Testergebnisse mit den erfindungsgemäßen Carbodiimiden (I) und (II) zeigen, dass diese im Vergleich zu den im Stand der Technik bekannten Carbodiimiden eine sehr hohe Wirksamkeit als Hydrolyseschutzmittel/Säurefänger in esterbasierten Polymeren und/oder in esterbasierten Formulierungen bei gleichzeitig sehr guter oder sogar verbesserter thermischen Stabilität aufweisen.

## Patentansprüche

1. Carbodiimide der Formel (I) 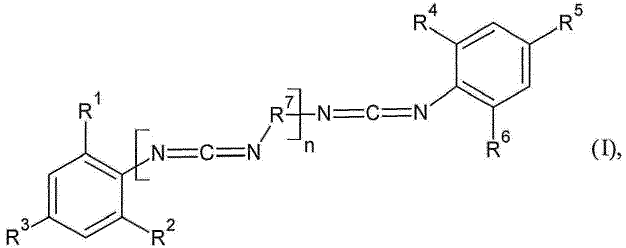 in der R¹, R², R³, R⁴, R⁵ und R⁶ unabhängig voneinander = C₁-C₂₀-Alkyl, C₃-C₂₀-Cycloalkyl, C₆-C₁₅-Aryl und/oder C₇-C₁₅-Aralkyl ist,
R⁷ = dreifach substituiertes C₁-C₄-alkyl-substituiertes Arylen und
n eine ganze Zahl von 4 bis 500, besonders bevorzugt von 4 - 50 ist.

2. Carbodiimide nach Anspruch 1, **dadurch gekennzeichnet, dass** die Reste R¹ bis R⁶ innerhalb des Moleküls gleich sind.

3. Carbodiimide nach Anspruch 1 oder 2, **dadurch gekennzeichnet, dass** die Reste R¹ bis R⁶ = Isopropyl sind.

4. Carbodiimide nach einem oder mehreren der Ansprüche 1 bis 3, **dadurch gekennzeichnet, dass** es sich um Mischungen aus mehreren Carbodiimiden handelt.

5. Carbodiimide nach einem oder mehreren der Ansprüche 1 bis 4, **dadurch gekennzeichnet, dass** es sich bei dem C₁-C₄-alkyl-substituierten Arylen um Triisopropylphenylen handelt.

6. Verfahren zur Herstellung der Carbodiimide nach einem oder mehreren der Ansprüche 1 bis 5, **dadurch gekennzeichnet, dass** trisubstituierte Phenylisocyanate der Formel (II) 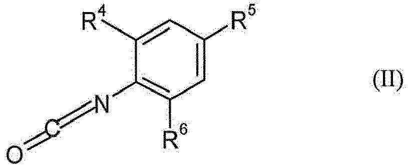 und der Formel (III) 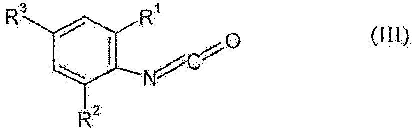 in der R¹, R², R³, R⁴, R⁵ und R⁶ unabhängig voneinander C₁-C₂₀-Alkyl, C₃-C₂₀-Cycloalkyl, C₆-C₁₅-Aryl und/oder C₇-C₁₅-Aralkyl ist,
und Verbindungen der Formel (IV) 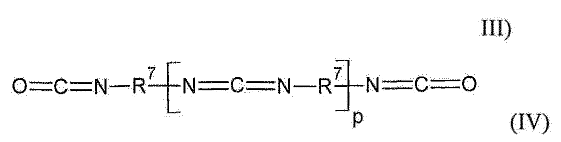 in der R⁷ ein dreifach substituiertes C₁-C₄-alkyl-substituiertes Arylen ist und p ≥ 2 darstellt,
unter Abspaltung von Kohlendioxid bei Temperaturen von 40 bis 200 °C in Gegenwart von Katalysatoren und gegebenenfalls eines Lösemittels carbodiimidisiert werden.

7. Verwendung der Carbodiimide nach einem oder mehreren der Ansprüche 1 bis 5 als Stabilisator, Vernetzer und/oder Kompatibilisator in Estergruppen enthaltenden Kunststoffen.

8. Verwendung der Carbodiimide nach einem oder mehreren der Ansprüche 1 bis 5 als Hydrolyseschutzmittel für esterbasierte Polyole, Polyurethan-Hart- und Weichschäume, estergruppen-enthaltende Polyurethanklebstoffe, Schmierstoffe und/oder Öle.

9. Verwendung der Carbodiimide nach einem oder mehreren der Ansprüche 1 bis 5 zur Feststoffdosierung auf kontinuierlich oder diskontinuierlich arbeitenden Verarbeitungsmaschinen.

10. Verwendung der Carbodiimide nach einem oder mehreren der Ansprüche 1 bis 5 zur Herstellung von Kunststofffolien, insbesondere PET-Folien, TPU-Folien und PLA-Folien.

## Claims

1. Carbodiimides of the formula (I) 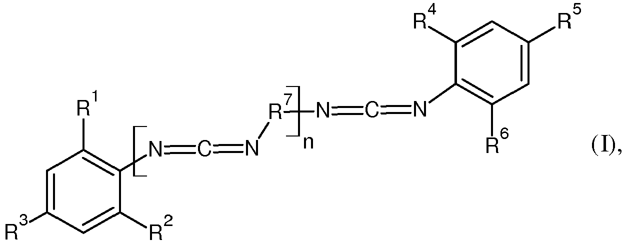 in which R¹, R², R³, R⁴, R⁵, and R⁶ independently of one another are each C₁-C₂₀-alkyl, C₃-C₂₀-cycloalkyl, C₆-C₁₅-aryl and/or C₇-C₁₅-aralkyl,
R⁷ is trisubstituted arylene substituted by C₁-C₄-alkyl, and
n is an integer from 4 to 500, particularly preferably from 4 - 50.

2. Carbodiimides according to Claim 1, **characterized in that** the radicals R¹ to R⁶ within the molecule are identical.

3. Carbodiimides according to Claim 1 or 2, **characterized in that** the radicals R¹ to R⁶ are isopropyl.

4. Carbodiimides according to one or more of Claims 1 to 3, **characterized in that** they are mixtures of two or more carbodiimides.

5. Carbodiimides according to one or more of Claims 1 to 4, **characterized in that** the arylene substituted by C₁-C₄-alkyl is triisopropylphenylene

6. Process for preparing the carbodiimides according to one or more of Claims 1 to 5, **characterized in that** trisubstituted phenyl isocyanates of the formula (II) 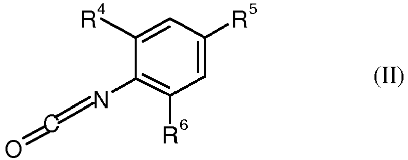 and of the formula (III) 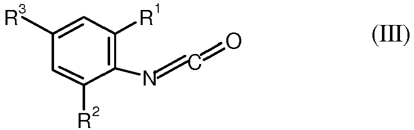 in which R¹, R², R³, R⁴, R⁵, and R⁶ independently of one another are each C₁-C₂₀-alkyl, C₃-C₂₀-cycloalkyl, C₆-C₁₅-aryl and/or C₇-C₁₅-aralkyl
and compounds of the formula (IV) 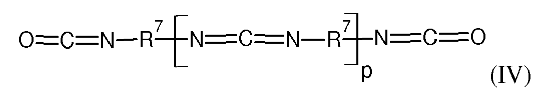 in which R⁷ is a trisubstituted arylene substituted by C₁-C₄-alkyl, and p represents ≥ 2,
are carbodiimidized with elimination of carbon dioxide at temperatures of 40 to 200°C in the presence of catalysts and optionally of a solvent.

7. Use of the carbodiimides according to one or more of Claims 1 to 5 as stabilizer, crosslinker and/or compatibilizer in plastics comprising ester groups.

8. Use of the carbodiimides according to one or more of Claims 1 to 5 as antihydrolysis agents for ester-based polyols, rigid and flexible polyurethane foams, ester group-comprising polyurethane adhesives, lubricants and/or oils.

9. Use of the carbodiimides according to one or more of Claims 1 to 5 for solids metering on continuously or discontinuously operating processing machines.

10. Use of the carbodiimides according to one or more of Claims 1 to 5 for producing plastics films, more particularly PET films, TPU films, and PLA films.

## Revendications

1. Carbodiimides de formule (I) 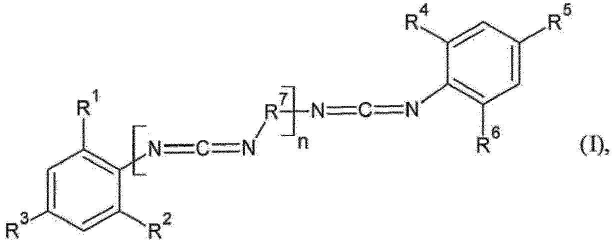 dans laquelle R¹, R², R³, R⁴, R⁵ et R⁶ représentent indépendamment les uns des autres alkyle en C₁-C₂₀, cycloalkyle en C₃-C₂₀, aryle en C₆-C₁₅ et/ou aralkyle en C₇-C₁₅,
R⁷ = arylène substitué trois fois par alkyle en C₁-C₄, et
n est un nombre entier de 4 à 500, de manière particulièrement préférée de 4 à 50.

2. Carbodiimides selon la revendication 1, **caractérisés en ce que** les radicaux R¹ à R⁶ dans la molécule sont identiques.

3. Carbodiimides selon la revendication 1 ou 2, **caractérisés en ce que** les radicaux R¹ à R⁶ = isopropyle.

4. Carbodiimides selon une ou plusieurs des revendications 1 à 3, **caractérisés en ce qu'**il s'agit de mélanges de plusieurs carbodiimides.

5. Carbodiimides selon une ou plusieurs des revendications 1 à 4, **caractérisés en ce que** l'arylène substitué par alkyle en C₁-C₄ est le triisopropylphénylène.

6. Procédé de fabrication des carbodiimides selon une ou plusieurs des revendications 1 à 5, **caractérisé en ce que** des isocyanates de phényle trisubstitués de formule (II) 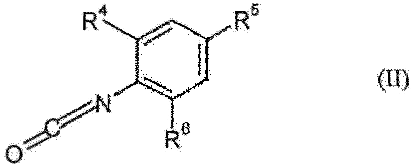 et de formule (III) 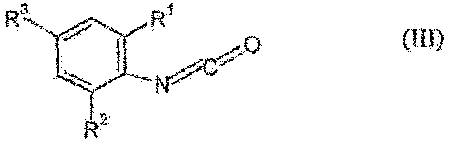 dans lesquelles R¹, R², R³, R⁴, R⁵ et R⁶ représentent indépendamment les uns des autres alkyle en C₁-C₂₀, cycloalkyle en C₃-C₂₀, aryle en C₆-C₁₅ et/ou aralkyle en C₇-C₁₅,
et des composés de formule (IV) 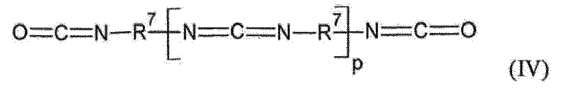 dans laquelle R⁷ représente un arylène substitué trois fois par alkyle en C₁-C₄ et p ≥ 2,
sont carbodiimidés avec clivage de dioxyde de carbone à des températures de 40 à 200 °C en présence de catalyseurs et éventuellement d'un solvant.

7. Utilisation des carbodiimides selon une ou plusieurs des revendications 1 à 5 en tant que stabilisateur, agent de réticulation et/ou compatibilisateur dans des plastiques contenant des groupes ester.

8. Utilisation des carbodiimides selon une ou plusieurs des revendications 1 à 5 en tant qu'agent anti-hydrolyse pour des polyols à base d'esters, des mousses dures et souples de polyuréthane, des adhésifs de polyuréthane contenant des groupes ester, des lubrifiants et/ou des huiles.

9. Utilisation des carbodiimides selon une ou plusieurs des revendications 1 à 5 pour le dosage de solides dans des machines d'usinage fonctionnant de manière continue ou discontinue.

10. Utilisation des carbodiimides selon une ou plusieurs des revendications 1 à 5 pour la fabrication de films plastiques, notamment de films de PET, de films de TPU et de films de PLA.
